(19) Europäisches Patentamt
European Patent Office
Office européen des brevets

(11) **EP 4 439 570 A1**

(12) **EUROPEAN PATENT APPLICATION**

(43) Date of publication:
**02.10.2024 Bulletin 2024/40**

(21) Application number: **24153929.5**

(22) Date of filing: **25.01.2024**

(51) International Patent Classification (IPC):
**G16C 20/50** *(2019.01)*    *G16C 20/30* *(2019.01)*
**G16C 20/70** *(2019.01)*

(52) Cooperative Patent Classification (CPC):
**G16C 20/50;** G06N 3/00; G16C 20/30; G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 IN 202321024704**

(71) Applicant: **Tata Consultancy Services Limited Maharashtra (IN)**

(72) Inventors:
• **BUNG, Navneet**
  **Hyderabad (IN)**

• **SRINIVASAN, Rajgopal**
  **500081 Hyerabad (IN)**
• **VANGALA, Sarveswararao**
  **500081 Hyderabad (IN)**
• **KRISHNAN, Sowmya Ramaswamy**
  **600113 Chennai (IN)**
• **ROY, Arijit**
  **500081 Hyderabad (IN)**

(74) Representative: **Goddar, Heinz J.**
  **Boehmert & Boehmert**
  **Anwaltspartnerschaft mbB**
  **Pettenkoferstrasse 22**
  **80336 München (DE)**

(54) **SYSTEM AND METHOD FOR EXTRACTION OF SMALL MOLECULE FRAGMENTS AND THEIR EXPLANATION FOR DRUG-LIKE PROPERTIES**

(57)    The embodiments of present disclosure herein address the inability of existing techniques to fragment both small molecules and substituents of a core scaffold. And it addresses generation of lesser number of unique fragments which hinders application of graph propagation approaches to predict properties from molecular datasets. The method and system for extraction of small molecule fragments and their explanation for drug-like properties. A molecular graph representation is used to train graph convolution network (GCN) models for prediction of various absorption, distribution, metabolism, excretion, and toxicity (ADMET) properties. The models developed are compared with an existing atom-level graph model trained using a similar architecture. Further, the explanations obtained from the predictive models are validated based on their relevance to the existing knowledgebase of substructure contributions using matched molecular pairs (MMP) analysis.

FIG. 2

**Description**

CROSS-REFERENCE TO RELATED APPLICATIONS AND PRIORITY

The present application claims priority from Indian patent application number 202321024704, filed on March 31, 2023.

TECHNICAL FIELD

The disclosure herein generally relates to the field of drug discovery and more specifically, to a method and system for extraction of small molecule fragments and their explanation for drug-like properties.

BACKGROUND

The advances in artificial intelligence (AI) algorithms have impacted drug discovery in many ways. It is possible to develop predictive AI models for various drug-like properties which can aid in accelerating drug discovery by screening unwanted molecules during the early stages of drug discovery. Understanding the reason behind model predictions can guide the medicinal chemist to identify fragments/substructures that can make the molecules undesirable during the lead optimization stage of drug discovery and modify them accordingly.

Although explanations from molecular graph representations (typically involving coloring schemes highlighting the magnitude of gradient change), are in good agreement with the experimental knowledgebase of medicinal chemists, these methods tend to produce partial explanations in molecules involving ring systems or functional groups. Most of the existing fragmentation methods such as Breaking of Retrosynthetically Interesting Chemical Substructures (BRICS), Retrosynthetic Combinatorial Analysis Procedure (RECAP), and Synthetic Disconnection Rules (SynDiR) lead to a singleton set upon fragmentation, cannot handle very small molecules, and fail to fragment all substituents of a core scaffold in a molecule. Specifically, some substituents such as halogen atoms, methyl and hydroxyl groups can be considered valid fragments of interest to medicinal chemists, due to the possibility of extensive change in the physicochemical and absorption, distribution, metabolism, excretion, and toxicity (ADMET) properties of a molecule upon their substitution. However, current explainable approaches are mostly atom-based where, often only a fraction of a fragment is shown to be significant.

SUMMARY

Embodiments of the disclosure present technological improvements as solutions to one or more of the above-mentioned technical problems recognized by the inventors in conventional systems. For example, in one embodiment, a method and system for extraction of small molecule fragments and their explanation for drug-like properties is provided.

In one aspect, a processor-implemented method for extraction of small molecule fragments and their explanation for drug-like properties is provided. The processor-implemented method includes one or more steps such as receiving a plurality of molecular representations of a molecule as an input. Further, the processor-implemented method comprising analyzing the received plurality of molecular representations to identify at least one technique for fragmentation of the received input, executing the identified at least one technique to fragment the received molecule in one or more substituents, and extracting a Murcko scaffold of each of the one or more substituents to identify one or more unmapped substituents from each of the one or more substituents. Further, the processor-implemented method comprising matching the one or more substituents against a predefined library of a ring and non-ring substituents to obtain a match of at least one unmapped substituent of the one or more substituents, generating a domain-aware graph of the received molecule using the obtained match of the least one unmapped substituent of the one or more substituents, and training a prediction model involving a deep learning model using the generated domain-aware graph of the received molecule, wherein the trained prediction model is used for one or more properties prediction. Further, the processor-implemented method comprising obtaining a node level contribution of the molecule towards the one or more properties using a Gradient Class Activation Maps (GradCAM) and finally the received molecule using the obtained node level contribution from the GradCAM analysis.

In another aspect, a system for extraction of small molecule fragments and their explanation for drug-like properties is provided. The system includes an input/output interface configured to take a plurality of molecular representations of a molecule as input, one or more hardware processors and at least one memory storing a plurality of instructions, wherein the one or more hardware processors are configured to execute the plurality of instructions stored in the at least one memory.

Further, the system is configured to analyze the received plurality of molecular representations to identify at least one technique for fragmenting the received input. The identified at least one technique is executed to fragment the received molecule in one or more substituents. Further, the system is configured to extract a Murcko scaffold of each of the one or more substituents to identify one or more unmapped substituents from each of the one or more substituents. The one or more substituents are matched against a predefined library of ring and non-ring substituents to obtain a match of at least one unmapped substituent of the one or more substituents. Further, the system is configured to generate a domain-aware graph of the received molecule using the obtained match of the least one unmapped substituent of the one or more substituents. A prediction model involving a deep learning model is trained with the generated domain-aware graph of the received molecule, wherein the trained prediction model is used for prediction of one or more properties. Furthermore, the system is configured to obtain a node level contribution of the molecule towards the one or more properties using a Gradient Class Activation Maps (GradCAM) and finally the received molecule is optimized using the obtained node level contribution from the GradCAM analysis.

In yet another aspect, one or more non-transitory machine-readable information storage mediums are provided comprising one or more instructions, which when executed by one or more hardware processors causes a method for extraction of small molecule fragments and their explanation for drug-like properties is provided. The processor-implemented method includes one or more steps such as receiving a plurality of molecular representations of a molecule as an input. Further, the processor-implemented method comprising analyzing the received plurality of molecular representations to identify at least one technique for fragmenting the received input, executing the identified at least one technique to fragment the received molecule in one or more substituents, and extracting a Murcko scaffold of each of the one or more substituents to identify one or more unmapped substituents from each of the one or more substituents. Further, the processor-implemented method comprising matching the one or more substituents against a predefined library of a ring and non-ring substituents to obtain a match of at least one unmapped substituent of the one or more substituents, generating a domain-aware graph of the received molecule using the obtained match of the least one unmapped substituent of the one or more substituents, and training a prediction model involving a deep learning model using the generated domain-aware graph of the received molecule, wherein the trained prediction model is used for prediction of one or more properties. Further, the processor-implemented method comprising obtaining a node level contribution of the molecule towards the one or more properties using a Gradient Class Activation Maps (GradCAM) and finally the received molecule using the obtained node level contribution from the GradCAM analysis.

It is to be understood that the foregoing general descriptions and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a network diagram of an exemplary system for extraction of small molecule fragments and their explanation for drug-like properties, in accordance with some embodiments of the present disclosure.
FIG. 2 is a functional block diagram of the system for extraction of small molecule fragments and their explanation for drug-like properties, in accordance with some embodiments of the present disclosure.
FIG. 3 is a flow diagram illustrating a method for extraction of small molecule fragments and their explanation for drug-like properties in accordance with some embodiments of the present disclosure.
FIGS. 4A and 4B are schematic diagrams to illustrate correctly predicted Matched Molecular Pairs (MMPs) using a multi-task model for BBB property, in accordance with some embodiments of the present disclosure.
FIGS. 5A and 5B are schematic diagrams to illustrate correctly predicted Matched Molecular Pairs (MMPs) using the multi-task model, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The advances in deep learning algorithms have impacted the drug discovery pipeline in many ways. Specifically, generative artificial intelligence (AI) algorithms can now explore the large chemical space and design novel and diverse molecules. While there has been significant progress in generative AI models, it is equally important to develop predictive models for various properties, which can help to characterize novel drug-like molecules. Further, the predictive model acts as a critic to design multi-property optimized molecules, which can potentially reduce the late stage attrition of drug candidates. Nevertheless, understanding the reason behind model predictions can guide the medicinal chemist to modify substructures that can make the molecules undesirable during the lead optimization stage of drug discovery. However, current explainable approaches are mostly atom-based where, often only a fraction of a fragment is shown to be significant.

The embodiments herein provide a method and system for extraction of small molecule fragments and their explanation for drug-like properties, in accordance with some embodiments of the present disclosure. The method and system provide a comprehensive solution to the problems with existing fragmentation techniques. It addresses the following problems:

1. Inability of existing techniques to fragment both small molecules and substituents of a core scaffold.
2. Generation of larger number of unique fragments which hinders application of graph propagation approaches to predict properties from molecular datasets.
3. Existing explainable techniques giving partial explanations in molecules involving ring systems.

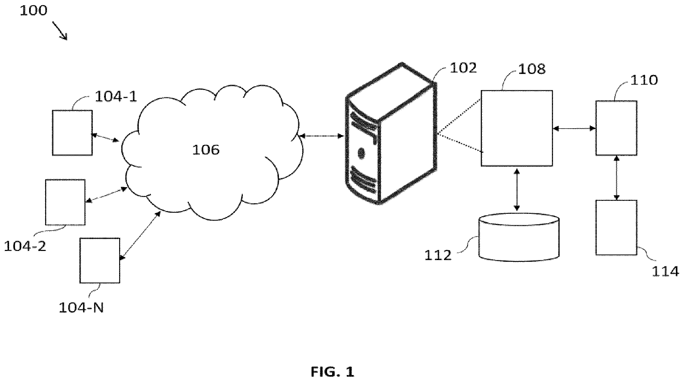

Referring now to the drawings, and more particularly to FIG. 1 through FIG. 5B, where similar reference characters denote corresponding features consistently throughout the figures, there are shown preferred embodiments, and these embodiments are described in the context of the following exemplary system and/or method.

FIG. 1 illustrates a block diagram of a system 100 for extraction of small molecule fragments and their explanation for drug-like properties, in accordance with an example embodiment. Although the present disclosure is explained considering that the system 100 is implemented on a server, it may be understood that the system 100 may comprise one or more computing devices 102, such as a laptop computer, a desktop computer, a notebook, a workstation, a cloud-based computing environment and the like. It will be understood that the system (100) may be accessed through one or more input/output interfaces 104-1, 104-2... 104-N, collectively referred to as I/O interface 104. Examples of the I/O interface 104 may include, but are not limited to, a user interface, a portable computer, a personal digital assistant, a handheld device, a smartphone, a tablet computer, a workstation, and the like. The I/O interface 104 are communicatively coupled to the system 100 through a network 106.

In an embodiment, the network 106 may be a wireless or a wired network, or a combination thereof. In an example, the network 106 can be implemented as a computer network, as one of the different types of networks, such as virtual private network (VPN), intranet, local area network (LAN), wide area network (WAN), the internet, and such. The network 106 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 106 may include a variety of network devices, including routers, bridges, servers, computing devices, storage devices. The network devices within the network 106 may interact with the system 100 through communication links.

The system 100 supports various connectivity options such as BLUETOOTH®, USB, ZigBee, and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. Further, the system 100 comprises at least one memory 110 with a plurality of instructions, one or more databases 112, and one or more hardware processors 108 which are communicatively coupled with the at least one memory to execute a plurality of modules 114 therein. The components and functionalities of the system 100 are described further in detail.

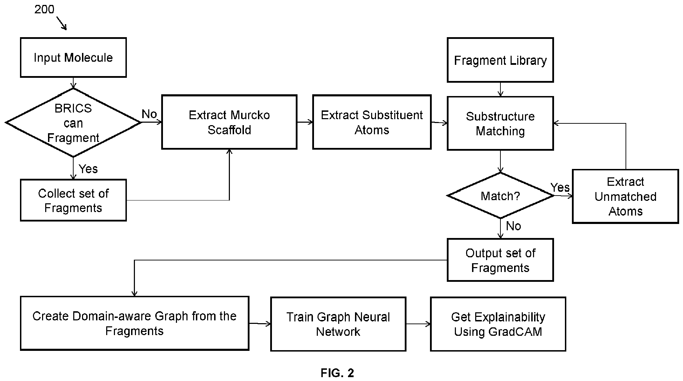

FIG. 2 is a functional block diagram 200 of the system 100 for extraction of small molecule fragments and their explanation for drug-like properties. The disclosure has addressed two challenges through the development of a molecular representation:

1. Training property prediction models on standardized datasets, and
2. Using gradient based explainability techniques to quantify fragment-level contribution to the property prediction.

A molecular fragmentation technique such as a post-processing Breaking of Retrosynthetically Interesting Chemical Substructures -pBRICS is developed for fine-grained fragmentation of small molecule. With the fragments of pBRICS, a molecular graph representation is used to train graph convolution network (GCN) models for prediction of different absorption, distribution, metabolism, excretion, and toxicity (ADMET) properties. Further, the explanations obtained from the predictive models are validated based on their relevance to the existing knowledgebase of substructure contributions using matched molecular pairs analysis.

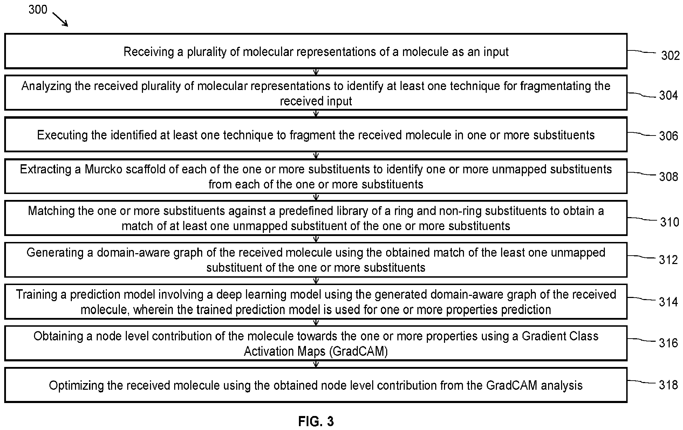

FIG. 3 is an exemplary flow diagram illustrating a processor-implemented method 300 for extraction of small molecule fragments and their explanation for drug-like properties implemented by the system of FIG. 1. Functions of the components of the system 100 are now explained with reference to FIG. 2 through steps of flow diagram in FIG. 3, according to some embodiments of the present disclosure.

Initially at step 302 of the processor-implemented method 300, a plurality of molecular representations of a molecule is received, via an input/output interface 104, as an input.

At the next step 304 of the processor-implemented method 300, the one or more hardware processors 108 are configured by the programmed instructions to analyze the received plurality of molecular representations to identify at least one technique for fragmentating the received input.

At the next step 306 of the processor-implemented method 300, the one or more hardware processors 108 are configured by the programmed instructions to execute the identified at least one technique to fragment the received molecule in one or more substituents, wherein each of the one or more substituents is a functional component of the received molecule.

A post-processing Breaking of Retrosynthetically Interesting Chemical Substructures (pBRICS) technique performs a post-processing of fragmentation results from the commonly used a Breaking of Retrosynthetically Interesting Chemical Substructures (BRICS) technique. The BRICS technique considers 16 chemical environments and their corresponding fragment prototypes for fragmentating of every bond in the received molecule. The pBRICS takes the output of the BRICS technique to further divide the fragments into finer set of fragments. The pBRICS attempts to iteratively fragment the molecule such that the smallest possible substituents of a scaffold are also enumerated, which can be of interest in building a knowledgebase for molecular optimization problems.

At the next step 308 of the processor-implemented method 300, the one or more hardware processors 108 are configured by the programmed instructions to extract a Murcko scaffold of each of the one or more substituents to identify one or more unmapped substituents from each of the one or more substituents.

It would be appreciated that the pBRICS technique uses a comprehensive library of fragments (ring systems and non-ring substituents) manually curated from an Open Chemistry project database and literature. If a molecule can be fragmented by the BRICS technique, the resultant fragments are primarily classified into scaffolds and substituents. The scaffold of a molecule or a fragment is defined based on the Bemis-Murcko frameworks (Murcko scaffold)(Bemis & Murcko (1996)) as implemented in a RDKit.

At the next step 310 of the processor-implemented method 300, the one or more hardware processors 108 are configured by the programmed instructions to match the one or more unmapped substituents against a predefined library of ring and non-ring substituents to obtain a match of the one or more unmapped substituents.

The atoms corresponding to substituents are further matched against the library of fragments to obtain the largest possible fragment match, and the process is iterated until no further fragment matches are possible. If a molecule cannot be fragmented by the BRICS method, the atoms corresponding to scaffold and substituents of the molecule are enumerated and the iterative fragmentation procedure is applied on the substituent atoms, instead of fragments from the BRICS technique. The pBRICS method is implemented in Python using utilities from the RDKit library.

At the next step 312 of the processor-implemented method 300, the one or more hardware processors 108 are configured by the programmed instructions to generate a domain-aware graph of the received molecule using the obtained match of the one or more unmapped substituents. Further, a feature vector is defined for each node of the domain-aware graph of the received molecule. The process of graph construction begins by taking the fragments as nodes and if two fragments are connected in the molecule by at least one bond, then an edge is formed between the corresponding nodes (fragments) in the graph.

For generating the graph, the one or more substituents are considered as nodes and an edge is defined between two nodes, if the two substituents are connected in the molecule by at least one bond. Once the graph construction is complete, the next step is to define the feature vector for each node in the graph.

At the next step 314 of the processor-implemented method 300, the one or more hardware processors 108 are configured by the programmed instructions to train a prediction model involving a deep learning model using the domain-aware graph of the received molecule. The trained prediction model is used for prediction of one or more properties. The domain-aware graph generated using the pBRICS fragmentation technique is used as input to the deep learning models involving Graph Convolutional Network (GCN) layers as implemented in the deep graph Library (DGL). The GCN-based architecture is being provided to yield promising results for predicting a wide range of molecular characteristics. Typically, the graph ($G$) is defined as:

$$G = (V, E) \qquad\qquad (1)$$

wherein fragments are nodes ($V$) and bond connecting them are edges ($E$). GCN layer modifies each node's embedding in the following way:

1. Using the node feature matrix $X = F^{n \times d}$ and an adj acency matrix $A \in \{0,1\}^{n \times n}$, GCN aggregates the information from the neighboring nodes.
2. Applies a non-linear activation function to the aggregated feature vector embedding of nodes to get the prediction.

In one aspect, a binary cross-entropy-loss (BCE) between the actual values and model predictions is calculated for each of the 23 classification tasks mentioned in Table 2. Herein, the loss is back propagated to update the weights of the neurons in the fully connected layers and the GCN layers. Area Under Receiver Operating System Curve (auROC) is used for measuring the performance of the classification models in this study.

$$BCE = \frac{-1}{N} \sum_{i=1}^{N} \left( y_i log(\hat{y}_i) + (1 - y_i) log (1 - \hat{y}_i) \right) \qquad (2)$$

wherein $N$ - number of samples, $y$ - predicted value and $\hat{y}_i$- true value.

At the next step 316 of the processor-implemented method 300, the one or more hardware processors 108 are configured by the programmed instructions to obtain a node level contribution of the molecule towards the one or more properties using a Gradient Class Activation Maps (GradCAM). Herein, the GradCAM calculates the node level scores.

Finally, at the last step 318 of the processor-implemented method 300, the one or more hardware processors 108 are configured by the programmed instructions to optimize the received molecule using the obtained node level contribution from the GradCAM analysis.

In one embodiment, consider $c$ to be the class for which explanations are generated, $L$ to be final GCN layer of the model, $\alpha_k^{L,c}$ to be the Grad-Cam weight for the $k^{th}$ feature of class $c$ at layer $L$, $N$ to be the number of nodes, and $F_k^{L,c}$ be the feature map of $k^{th}$ feature map at layer $L$ of node $n$:

$$\alpha_k^{L,c} = \frac{1}{N} \sum_{n=1}^{N} \frac{\partial y^c}{\partial F_{k,n}^L} \qquad (3)$$

Node level importance scores for a graph obtained using the Grad-CAM technique is given by equation 3. Based on these scores, one can identify the fragments (nodes) contributing positively to the class $c$.

$$L_{Grad-CAM}^c[L, n] = \sum_k \alpha_k^{L,c} F_{k,n}^L(X, A) \qquad (4)$$

The pBRICS fragmentation technique is compared with the existing fragmentation techniques namely, Breaking of Retrosynthetically Interesting Chemical Substructures (BRICS), Retrosynthetic Combinatorial Analysis Procedure (RECAP), and Synthetic Disconnection Rules (SynDiR) in terms of four metrics defined earlier with the ChEMBL dataset as the benchmark dataset as shown in Table 1.

**Table 1**

| Metric | pBRICS | BRICS | RECAP | SynDiR |
|---|---|---|---|---|
| No. of uncut molecules | 5,908 | 55,677 | 152,177 | 97,823 |
| Total no. of fragments generated (with duplicates) | 11,913,61 | 8,328,962 | 4,964,387 | 5,672,031 |
| No of unique fragments | 120,552 | 242,208 | 242,208 | 342, 263 |
| Average heavy atom count for the unique fragments | 12,201 | 15,593 | 15,911 | 17,693 |

Based on the comparison result it is notable that the pBRICS generates the least number of unique fragments with smallest heavy atom count, resulting in a more generic fragment library due to the fine-grained fragmentation procedure developed. Further, the pBRICS can fragment 16-times, 5-times, and 10-times more molecules that RECAP, BRICS and SyDiR techniques respectively, when benchmarked on the ChEMBL dataset of ~1.6 million drug-like small molecules.

To validate the pBRICS technique, a Matched Molecular Pairs (MMP) dataset is curated for each property. However, except for blood brain barrier permeability (BBBP) and Ames mutagenicity, there are not many MMP pairs for rest of the properties.

The challenge for the predictive model is to accurately predict small molecules that belong to the MMP dataset and are not part of the same class. The MMP dataset for a blood brain barrier permeability (BBBP) property consists of 102 unique pairs of molecules along with their corresponding ground-truth labels. To check the model performance on the MMP dataset, the molecules from all pairs of MMP entries were combined to form a unique set of 110 molecules.

Further, a Gradient Class Activation Map (Grad-CAM) algorithm is applied on all the molecules in the MMP dataset to get the gradient weights corresponding to each node (fragment) in the graph. Correct predictions are defined as cases where the positively labelled molecules obtain a positive gradient or weight for the corresponding MMP transformation identified and vice versa.

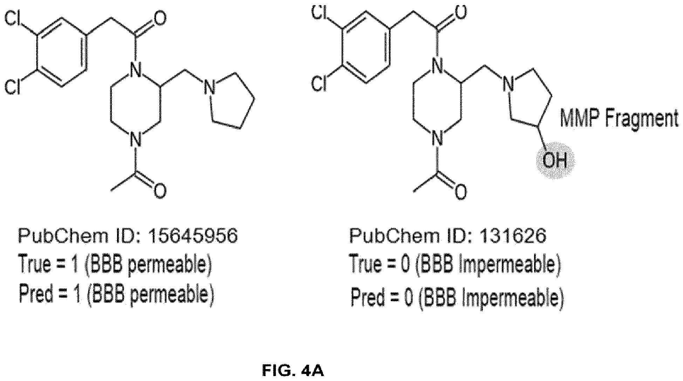

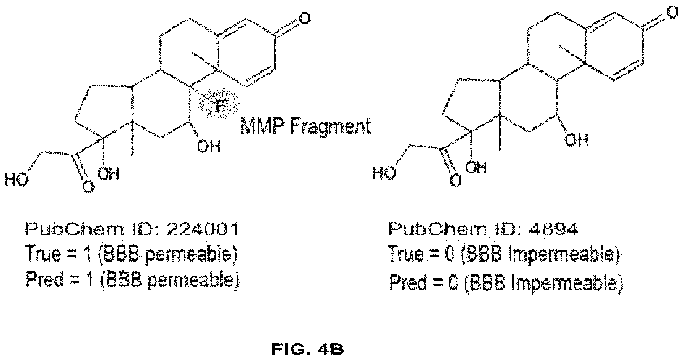

FIGS. 4A and 4B depict two examples of correctly predicted MMP pairs from the dataset, whose Grad-CAM values are found to match with their labels. In FIG. 4A, the MMP adds a hydroxyl group to the molecule and the molecule is transformed from BBB permeable to BBB impermeable. In another case as shown in FIG. 4B, replacing the halogen (Fluorine) with hydrogen atom transformed the molecules from BBB permeable to BBB impermeable. It is notable that these transformations are also experimentally proven to disrupt BBBP property of a molecule.

For each MMP transformation present in the BBBP dataset, the number of MMP pairs with matching model predictions were identified and filtered to extract entries with at least 5 matches, which are considered to be significant. Here, the transformation of methyl to Chlorine, was observed to change the label from impermeable to permeable. If the mean label of a transformation was positive, then the corresponding transformation was considered to have a significant effect in transforming the impermeable molecules to permeable molecules and vice versa.

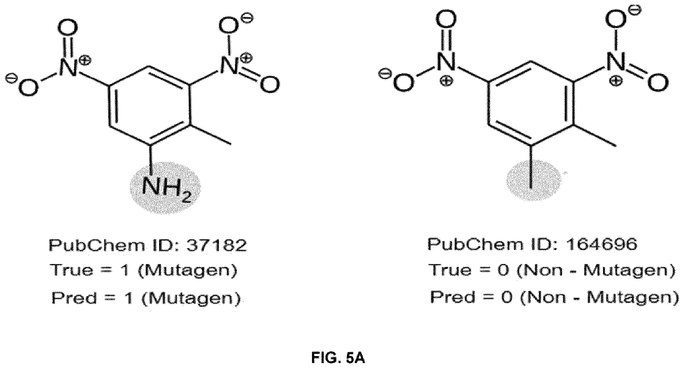

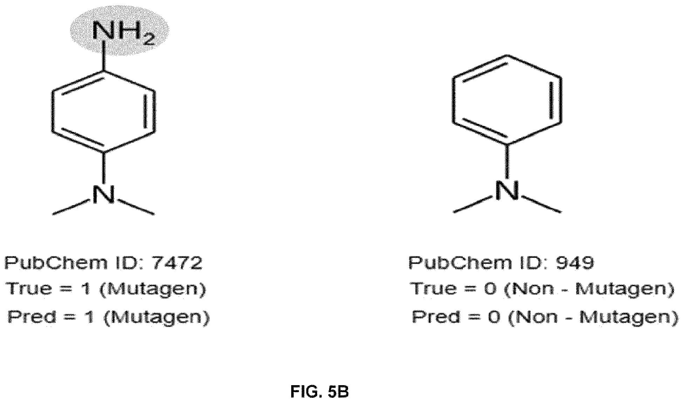

A similar analysis of MMP dataset is performed for Ames mutagenicity property as shown in FIGS. 5A and 5B. The Ames mutagenicity consists of 600 unique pairs of molecules along with their corresponding ground-truth labels. To check the model performance on the MMP dataset, the molecules from all pairs of MMP entries are combined to form a unique set of 712 molecules. The MMPs identified are supplied to both trained models and one external model similar to the BBBP property model, to obtain the predictions and corresponding results as shown in Table 2.

**Table 2**

| Task | Model | Performance |
|---|---|---|
| Combined SMILES list of all pairs of MMPs | MT-FraGCN | 433/712 |
| | ADMETLab 2.0 | 436/712 |
| | SwissADME | 405/712 |
| Matching both labels of MMPs | MT-FraGCN | 135/600 |
| | ADMETLab 2.0 | 98/600 |
| | SwissADME | 33/102 |

It is observed that Multi-task Fragment based GCN (MT-FraGCN) correctly predicts maximum MMP pairs. Based on Grad-CAM analysis, replacing an amino group with methyl group in the molecule transformed the molecule from mutagen to non-mutagen as shown in FIG. 5A, and replacing an amino with hydrogen atom transformed the molecules from mutagen to non-mutagen as shown in FIG. 5B.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address problem in the current explainable approaches those are mostly atom-based where, often only a fraction of a fragment is shown to be significant. The embodiments herein provide a method and system for extraction of small molecule fragments and their explanation for drug-like properties, in accordance with some embodiments of the present disclosure. The method and system provide a comprehensive solution to the problems with existing fragmentation techniques.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software modules located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

**Claims**

1. A processor-implemented method (300) comprising:

   receiving (302), via an input/output interface, a plurality of molecular representations of a molecule as an input;
   analyzing (304), via one or more hardware processors, the received plurality of molecular representations to identify at least one technique for fragmentating the received input;
   executing (306), via the one or more hardware processors, the identified at least one technique to fragment the received molecule in one or more substituents, wherein each of the one or more substituents is a functional component of the received molecule;
   extracting (308), via the one or more hardware processors, a Murcko scaffold of each of the one or more substituents to identify one or more unmapped substituents from each of the one or more substituents;
   matching (310), via the one or more hardware processors, the one or more substituents against a predefined library of a ring and non-ring substituents to obtain a match of at least one unmapped substituent of the one or more substituents;
   generating (312), via the one or more hardware processors, a domain-aware graph of the received molecule using the obtained match of the least one unmapped substituent of the one or more substituents;
   training (314), via the one or more hardware processors, a prediction model involving a deep learning model using the generated domain-aware graph of the received molecule, wherein the trained prediction model is used for one or more properties prediction;
   obtaining (316), via the one or more hardware processors, a node level contribution of the molecule towards the one or more properties using a Gradient Class Activation Maps (GradCAM), wherein the GradCAM calculates the node level scores; and
   optimizing (318), via the one or more hardware processors, the received molecule using the obtained node level contribution from the GradCAM analysis.

2. The processor-implemented method (300) as claimed in claim 1, wherein a feature vector is defined for each node of the generated domain-aware graph of the received molecule.

3. The processor-implemented method (300) as claimed in claim 1, wherein the training can be using one or more single-task and multi-task machine learning models.

4. The processor-implemented method (300) as claimed in claim 3, wherein the one or more single-task and multi-task machine learning models have a highest value of performance compared to existing models trained on the same dataset.

5. The processor-implemented method (300) as claimed in claim 1, wherein the plurality of molecular representations is obtained by representing a plurality of small molecules using the generated domain-aware graph.

6. A system (100) comprising:

at least one memory (110) storing a plurality of instructions;
one or more Input/Output (I/O) interfaces (104); and
one or more hardware processors (108) coupled to the memory via the one or more I/O interfaces, wherein the one or more hardware processors (108) are configured by the plurality of instructions to:

analyze the received plurality of molecular representations to identify at least one technique for fragmentating the received input;
execute the identified at least one technique to fragment the received molecule in one or more substituents, wherein each of the one or more substituents is a functional component of the received molecule;
extract a Murcko scaffold of each of the one or more substituents to identify one or more unmapped substituents from each of the one or more substituents;
match the one or more substituents against a predefined library of a ring and non-ring substituents to obtain a match of at least one unmapped substituent of the one or more substituents;
generate a domain-aware graph of the received molecule using the obtained match of the least one unmapped substituent of the one or more substituents;
train a prediction model involving a deep learning model using the generated domain-aware graph of the received molecule, wherein the trained prediction model is used for one or more properties prediction;
obtain a node level contribution of the molecule towards the one or more properties using a Gradient Class Activation Maps (GradCAM), wherein the GradCAM calculates the node level scores; and
optimize the received molecule using the obtained node level contribution from the GradCAM analysis.

7. The system (100) as claimed in claim 6, wherein a feature vector is defined for each node of the generated domain-aware graph of the received molecule.

8. The system (100) as claimed in claim 6, wherein the training can be using one or more single-task and multi-task machine learning models.

9. The system (100) as claimed in claim 8, wherein the one or more single-task and multi-task machine learning models have highest value of performance compared to existing models trained on the same dataset.

10. The system (100) as claimed in claim 6, wherein the plurality of molecular representations is obtained by representing a plurality of small molecules using the generated domain-aware graph.

11. One or more non-transitory machine-readable information storage mediums comprising one or more instructions which when executed by one or more hardware processors cause:

receiving, via an input/output interface, a plurality of molecular representations of a molecule as an input;
analyzing the received plurality of molecular representations to identify at least one technique for fragmentating the received input;
executing the identified at least one technique to fragment the received molecule in one or more substituents, wherein each of the one or more substituents is a functional component of the received molecule;
extracting a Murcko scaffold of each of the one or more substituents to identify one or more unmapped substituents from each of the one or more substituents;
matching the one or more substituents against a predefined library of a ring and non-ring substituents to obtain a match of at least one unmapped substituent of the one or more substituents;
generating a domain-aware graph of the received molecule using the obtained match of the least one unmapped substituent of the one or more substituents;

training a prediction model involving a deep learning model using the generated domain-aware graph of the received molecule,

wherein the trained prediction model is used for one or more properties prediction;

obtaining a node level contribution of the molecule towards the one or more properties using a Gradient Class Activation Maps (GradCAM), wherein the GradCAM calculates the node level scores; and

optimizing the received molecule using the obtained node level contribution from the GradCAM analysis.

12. The one or more non-transitory machine-readable information storage mediums as claimed in claim 11, wherein a feature vector is defined for each node of the generated domain-aware graph of the received molecule.

13. The one or more non-transitory machine-readable information storage mediums as claimed in claim 11, wherein the training can be using one or more single-task and multi-task machine learning models.

14. The one or more non-transitory machine-readable information storage mediums as claimed in claim 13, wherein the one or more single-task and multi-task machine learning models have highest value of performance compared to existing models trained on the same dataset.

15. The one or more non-transitory machine-readable information storage mediums as claimed in claim 11, wherein the plurality of molecular representations is obtained by representing a plurality of small molecules using the generated domain-aware graph.

**FIG. 1**

FIG. 2

300

| | |
|---|---|
| Receiving a plurality of molecular representations of a molecule as an input | 302 |
| Analyzing the received plurality of molecular representations to identify at least one technique for fragmentating the received input | 304 |
| Executing the identified at least one technique to fragment the received molecule in one or more substituents | 306 |
| Extracting a Murcko scaffold of each of the one or more substituents to identify one or more unmapped substituents from each of the one or more substituents | 308 |
| Matching the one or more substituents against a predefined library of a ring and non-ring substituents to obtain a match of at least one unmapped substituent of the one or more substituents | 310 |
| Generating a domain-aware graph of the received molecule using the obtained match of the least one unmapped substituent of the one or more substituents | 312 |
| Training a prediction model involving a deep learning model using the generated domain-aware graph of the received molecule, wherein the trained prediction model is used for one or more properties prediction | 314 |
| Obtaining a node level contribution of the molecule towards the one or more properties using a Gradient Class Activation Maps (GradCAM) | 316 |
| Optimizing the received molecule using the obtained node level contribution from the GradCAM analysis | 318 |

FIG. 3

PubChem ID: 15645956
True = 1 (BBB permeable)
Pred = 1 (BBB permeable)

PubChem ID: 131626
True = 0 (BBB Impermeable)
Pred = 0 (BBB Impermeable)

**FIG. 4A**

PubChem ID: 224001
True = 1 (BBB permeable)
Pred = 1 (BBB permeable)

PubChem ID: 4894
True = 0 (BBB Impermeable)
Pred = 0 (BBB Impermeable)

FIG. 4B

FIG. 5A

PubChem ID: 7472
True = 1 (Mutagen)
Pred = 1 (Mutagen)

PubChem ID: 949
True = 0 (Non - Mutagen)
Pred = 0 (Non - Mutagen)

FIG. 5B

## EUROPEAN SEARCH REPORT

Europäisches
Patentamt
European
Patent Office
Office européen
des brevets

Application Number

EP 24 15 3929

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GHERSI D. ET AL: "molBLOCKS: decomposing small molecule sets and uncovering enriched fragments", BIOINFORMATICS, vol. 30, no. 14, 28 March 2014 (2014-03-28), pages 2081-2083, XP093187885, GB ISSN: 1367-4803, DOI: 10.1093/bioinformatics/btu173 Retrieved from the Internet: URL:https://academic.oup.com/bioinformatics/article-pdf/30/14/2081/48925037/bioinformatics_30_14_2081.pdf> * the whole document * ----- | 1-15 | INV. G16C20/50 ADD. G16C20/30 G16C20/70 |
| X | ZHU W. ET AL: "HiGNN: A Hierarchical Informative Graph Neural Network for Molecular Property Prediction Equipped with Feature-Wise Attention", JOURNAL OF CHEMICAL INFORMATION AND MODELING, vol. 63, no. 1, 15 December 2022 (2022-12-15), pages 43-55, XP093187881, US ISSN: 1549-9596, DOI: 10.1021/acs.jcim.2c01099 Retrieved from the Internet: URL:https://pubs.acs.org/doi/pdf/10.1021/acs.jcim.2c01099> * the whole document * ----- -/-- | 1-15 | TECHNICAL FIELDS SEARCHED (IPC) G16C G06N |

The present search report has been drawn up for all claims

| Place of search | Date of completion of the search | Examiner |
|---|---|---|
| The Hague | 22 July 2024 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

# EP 4 439 570 A1

Europäisches Patentamt
European Patent Office
Office européen des brevets

## EUROPEAN SEARCH REPORT

**Application Number**

EP 24 15 3929

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Vangala S. R. ET AL: "DOMAIN-AWARE REPRESENTATION OF SMALL MOLECULES FOR EXPLAINABLE PROPERTY PREDIC-TION MODELS", ICLR 2023-Machine Learning for Drug Discovery workshop, 6 March 2023 (2023-03-06), XP093187883, Retrieved from the Internet: URL:https://openreview.net/forum?id=C9WW17 wQF7p * the whole document * | 1-15 | |
| X | POLISHCHUK P.: "CReM: chemically reasonable mutations framework for structure generation", JOURNAL OF CHEMINFORMATICS, vol. 12, no. 1, 28, 22 April 2020 (2020-04-22), XP093187884, London, UK ISSN: 1758-2946, DOI: 10.1186/s13321-020-00431-w Retrieved from the Internet: URL:https://link.springer.com/article/10.1 186/s13321-020-00431-w/fulltext.html> * the whole document * | 1-15 | TECHNICAL FIELDS SEARCHED (IPC) |

The present search report has been drawn up for all claims

| Place of search | Date of completion of the search | Examiner |
|---|---|---|
| The Hague | 22 July 2024 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321024704